# FASCICULE DE BREVET EUROPEEN

(11) **EP 1 438 950 B1**
(45) Date de publication et mention de la délivrance du brevet: **24.02.2010**
(21) Numéro de dépôt: 04290073.8
(22) Date de dépôt: 12.01.2004
(51) Int. Cl.: A61K 8/22, A61K 8/81, A61Q 5/08

(54) **Compositions de décoloration des cheveux**
Haarbleichmittel
Hair bleaching compositions

(30) Priorité: 16.01.2003 FR 0300455
(43) Date de publication de la demande: 21.07.2004
(73) Titulaire: L'Oréal, 75008 Paris (FR)
(72) Inventeur: Legrand, Frédéric, 92400 Courbevoie (FR)
(74) Mandataire: Wattremez, Catherine

(56) Documents cités:
- EP-A- 0 882 444
- EP-A- 1 036 558
- WO-A-02/00177
- US-A1- 2002 157 191
- R.C. PEPE, J.A. WENNINGER, G.N. MCEWEN: "International Cosmetic Ingredient Dictionary and Handbook" 2002 , THE COSMETIC, TOILETRY AND FRAGRANCE ASSOCIATION , WASHINGTON, D.C. XP002257246 9ème édition 2002, Vol. 1, p. 28-32 ISBN 1-882621-29-8 * le document en entier *

## Description

La présente invention a pour objet une composition de décoloration de fibres kératiniques, un procédé de décoloration de fibres kératiniques la mettant en oeuvre ainsi qu'un dispositif à plusieurs compartiments la comprenant.

Il est connu de décolorer des fibres kératiniques humaines, et en particulier les cheveux, en utilisant des composition de décoloration comprenant un ou plusieurs agents oxydants. Parmi les agents oxydants classiquement utilisés, on peut citer le peroxyde d'hydrogène ou des composés susceptibles de produire du peroxyde d'hydrogène par hydrolyse, comme par exemple l'urée ou les persels tels que les perborates, les persulfates, les percarbonates.
A l'origine, les compositions de décoloration se présentaient sous la forme de poudre. Elles avaient cependant l'inconvénient de produire de la poussière durant leur manutention, leur transport et leur stockage. De plus, ce phénomène était aggravé par le fait que les produits qui composent ces poudres sont corrosifs, irritants pour les yeux, pour les voies respiratoires et les muqueuses. C'est pourquoi, afin de résoudre les problèmes rencontrés avec l'usage de compositions pulvérulentes, on a récemment développé compositions de décoloration sous forme de pâte. Ainsi, les composés pulvérulents sont dispersés dans un support liquide inerte organique épaissi.
Si cette galénique apporte une solution aux problèmes de volatilité évoqués ci-dessus, la mise en oeuvre des compositions sous forme de pâte occasionne de nouvelles difficultés.

Ainsi, les compositions de décoloration, quelles soient sous forme de poudre ou de pâte, sont à mélanger avant emploi, à des compositions aqueuses de peroxyde d'hydrogène pour obtenir la composition de décoloration prête à l'emploi.

Ces compositions aqueuses de peroxyde d'hydrogène, sont sous forme de solutions aqueuses ou d'émulsions huile dans eau et sont plus ou moins liquides ou fluides.
Cette galénique favorise les mélanges avec des compositions de décoloration sous forme de poudre, dans la mesure où plus la composition aqueuse de peroxyde d'hydrogène est liquide ou fluide, plus la poudre décolorante se solubilise rapidement et facilement.
En revanche, les compositions de décoloration sous forme de pâte sont dépourvues d'eau et leur texture est compacte et dure. De plus, ces pâtes décolorantes sont à tendance hydrophobe compte tenu de la présence d'un taux élevé de liquide inerte organique. En conséquence, le mélange de la composition de décoloration et de la composition de peroxyde d'hydrogène est loin d'être facilité. Cela se traduit non seulement par un temps de mélange plus long mais aussi par une complication des opérations pour obtenir un mélange homogène.

L'une des solutions envisagées a été d'enrichir les émulsions huile dans eau de peroxyde d'hydrogène, de corps gras tels des alcools gras, afin d'obtenir des textures crèmes plus compactes. Toutefois, le différentiel de texture par rapport aux pâtes décolorantes anhydres reste important, et les mélanges sont toujours relativement longs à réaliser.

Il convient donc de trouver des compositions aqueuses oxydantes particulières dont le mélange avec des pâtes décolorantes soit plus rapide et plus facile.

La présente invention a pour objet de résoudre les problèmes mentionnés ci-dessus.

Ainsi, elle a pour objet une composition de décoloration de fibres kératiniques humaines, et en particulier des cheveux, prête à l'emploi, susceptible d'être obtenue en mélangeant avant emploi i) une composition de décoloration anhydre sous forme de pâte comprenant au moins un sel peroxygéné, au moins un agent alcalin et de 15 à 35 % d'au moins un liquide inerte organique, avec ii) une composition oxydante sous forme d'une émulsion huile dans eau de peroxyde d'hydrogène, comprenant au moins un tensioactif non ionique et/ou anionique et au moins un copolymère comprenant au moins un motif provenant d'un acide (méth)acrylique salifié ou non et au moins un motif provenant d'un ester d'acide (méth)acrylique avec un alcool gras en C₈-C₃₀, saturé ou insaturé, linéaire ou ramifié, éthoxylé.

Elle a de plus pour objet un procédé de préparation de ladite composition, consistant à mélanger avant emploi ladite composition de décoloration anhydre sous forme de pâte avec ladite composition oxydante sous forme d'une émulsion huile dans eau de peroxyde d'hydrogène.

Un autre objet de l'invention concerne un procédé de décoloration de fibres kératiniques humaines, en particulier des cheveux, consistant à appliquer la composition de décoloration prête à l'emploi selon l'invention, sur la zone des fibres kératiniques humaines, sèches ou humides, à décolorer ; à la laisser agir pendant un temps de pause suffisant pour obtenir la décoloration recherchée ; à éliminer la composition par un rinçage à l'eau, suivi d'un lavage avec un shampooing, puis éventuellement d'un séchage.

Enfin, l'invention concerne un dispositif à plusieurs compartiments, ou "kit", pour la mise en oeuvre du procédé de décoloration précité, comportant au moins deux compartiments dont l'un contient une composition de décoloration anhydre sous forme de pâte comprenant au moins un sel peroxygéné, au moins un agent alcalin et de 15 à 35 % d'au moins un liquide inerte organique ; et l'autre contient une émulsion huile dans eau de peroxyde d'hydrogène, comprenant au moins un tensioactif non ionique et/ou anionique et au moins un copolymère comprenant au moins un motif provenant d'un acide (méth)acrylique salifié ou non et au moins un motif provenant d'un ester d'acide (méth)acrylique avec un alcool gras C₈-C₃₀, saturé ou non, linéaire ou ramifié, éthoxylé.

Il a été trouvé que les mélanges de composition de décoloration anhydre sous forme de pâte avec des émulsions H/E de peroxyde d'hydrogène comprenant au moins un tensioactif non ionique et/ou anionique et un copolymère tel que décrit ci-dessus, pouvaient être atteints de manière significativement plus rapide, et de manière plus aisée.

De plus, les compositions de décoloration prêtes à l'emploi selon l'invention s'appliquent facilement et rapidement. Elles présentent une très bonne adhérence et ne coulent pas hors des zones que l'on souhaite décolorer.

Elles permettent enfin des décolorations puissantes et homogènes, tout en offrant de très bonnes propriétés cosmétiques.

Mais d'autres caractéristiques et avantages de la présente invention apparaîtront plus clairement à la lecture de la description et des exemples qui vont suivre.

La composition de décoloration anhydre va tout d'abord être décrite.

Dans ce qui va suivre, lorsque des gammes sont définies comme comprises entre deux valeurs, ces valeurs sont incluses.

### Composition de décoloration anhydre

Comme indiqué auparavant, ladite composition anhydre est sous forme de pâte. Plus particulièrement, cette pâte présente une teneur en eau inférieure à 1% en poids, et de préférence inférieure à 0,5% en poids par rapport au poids total de la pâte.

### Sel peroxygénés

Par ailleurs, la composition de décoloration anhydre comprend au moins un sel peroxygéné, qui est plus spécialement choisi parmi les persulfates, perborates, percarbonates, et peroxydes de métaux alcalins ou alcalino-terreux, comme le sodium, le potassium, le magnésium.
De préférence, la pâte comprend, à titre de sels peroxygénés, des persulfates, et préférence des persulfates de sodium et de potassium, seuls ou en mélange.

Habituellement, la teneur en sel peroxygéné dans la composition de décoloration anhydre est comprise entre 10 et 70 % en poids, de préférence entre 20 et 60 % en poids. Il est à noter que de manière avantageuse, la teneur en sel peroxygéné dans la composition prête à l'emploi est comprise entre 5 et 35 % en poids de la composition prête à l'emploi (c'est-à-dire comprenant le mélange de la composition de décoloration et la composition oxydante), de préférence entre 10 et 30 % en poids de la composition prête à l'emploi.

### Agents alcalins

La composition de décoloration anhydre comprend en outre au moins un agent alcalin qui est usuellement choisi parmi l'urée ; les sels d'ammonium, comme les chlorures, les sulfates, les phosphates, les nitrates ; les silicates, les phosphates, les carbonates de métaux alcalins (tels que le sodium, le potassium) ou alcalino-terreux (comme notamment le magnésium) seuls ou combinés.

Habituellement, la teneur en agent alcalin dans la composition de décoloration anhydre est comprise entre 0,01 et 40 % en poids, de préférence entre 0,1 et 30 % en poids. Selon un mode de réalisation particulier de l'invention, la teneur en agent alcalin dans la composition prête à l'emploi est comprise entre 0,005 et 20 % en poids, de préférence entre 0,05 et 15 % en poids.

### Liquide inerte

La composition de décoloration anhydre comprend enfin de 15 à 35 % en poids d'au moins un liquide inerte organique.
Par liquide, on entend un composé ou un mélange de composés liquides à 25°C et à pression atmosphérique.

Avantageusement, le liquide inerte organique est choisi parmi les polydécènes, les mono- et poly- esters d'acides carboxyliques, les mono- ou poly- esters de sucres d'acides en C₈-C₃₀, les éthers cycliques, les esters cycliques, les huiles silicones, les huiles minérales, les huiles végétales, ou leurs mélanges.

Plus particulièrement, les polydécènes sont des composés de formule C₁₀ₙH[(20n)+2] avec n variant de 3 à 9, et de préférence de 3 à 7. Ces composés répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.
On peut citer, à titre d'exemple, le produit vendu sous la dénomination Silkflo^{®} 366 NF Polydecene par la société Amoco Chemical, ceux vendus sous la dénomination Nexbase® 2002 FG, 2004 FG, 2006 FG et 2008 FG par la société Fortum.

En ce qui concerne les mono- ou poly- esters d'acides carboxyliques, ces derniers, linéaires ou ramifiés, saturés ou non, comprennent avantageusement au moins une chaîne hydrocarbonée en C₈-C₃₀, plus particulièrement en C₈-C₂₄, de préférence C₁₂-C₂₄, provenant de la partie acide ou alcool, et au moins une chaîne en C₁-C₈, de préférence en C₁-C₆. De plus, si l'acide carboxylique comprend plusieurs fonctions carboxyliques, celles-ci sont de préférence toutes estérifiées. Enfin, il est à noter que les alcools sont de préférence des alcools monofonctionnels.

A titre d'exemples, on peut citer les esters des acides uiéique, iaurique, palmitique, myristique, béhénique, stéarique, linoléique, linolénique, caprique, arachidonique, ou leurs mélanges comme notamment les mélanges oléo-palmitique, oléo-stéarique, palmito-stéarique, etc.
On peut de plus citer le diester isopropylique de l'acide sébacique (sébaçate de diisopropyle), l'adipate de di-octyle et le maléate de di-caprylyle.

On peut de plus utiliser un polyester d'un acide polycarboxylique comprenant un radical avec moins de 6 atomes de carbone, saturé ou non, linéaire ou ramifié, et d'un alcool comprenant un radical avec moins de 6 atomes de carbone, saturé ou non, linéaire ou ramifié. A titre d'exemple, on peut citer le citrate de triéthyle.

De préférence, les esters sont choisis parmi ceux obtenus à partir d'acides gras en C₁₂-C₂₄, plus particulièrement comprenant un groupement carboxylique, et d'un monoalcool saturé, linéaire ou ramifié, en C₃-C₆.
Selon une variante particulièrement avantageuse de l'invention, la composition de décoloration comprend, à titre de liquide inerte, le palmitate d'isopropyle, et de préférence le myristate d'isopropyle, seuls ou en mélanges.

Pour ce qui a trait aux mono- ou poly- esters de sucres d'acides en C₈-C₃₀, de préférence en C₁₂-C₂₄, il est précisé que le terme sucre désigne des composés qui possèdent plusieurs fonctions hydroxyle, avec ou sans fonction aldéhyde ou cétone, et qui comportent au moins 4 atomes de carbone. Ces sucres peuvent être des monosaccharides, des oligosaccharides ou des polysaccharides. Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.
Quant aux acides en en C₈-C₃₀, saturés ou non, linéaires ou ramifiés, comprenant une ou deux fonctions carboxyliques, on pourra se référer aux listes données auparavant.
Les esters peuvent être choisis parmi les mono-, di-, tri- et tétra-esters, les polyesters et leurs mélanges.
Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.
On peut citer, à titre d'exemples, le produit vendu sous la dénomination Glucate DO par la société Amerchol, qui est un dioléate de méthylglucose, les produits vendus sous les dénominations F160, F140, F110, F90, F70, SL40 par la société Crodesta, désignant respectivement les palmito-stéarates de sucrose formés de 73% de monoester et 27% de di- et tri-ester, de 61% de monoester et 39% de di-, tri-, et tétra-ester, de 52% de monoester et 48% de di-, tri-, et tétra-ester, de 45% de monoester et 55% de di-, tri-, et tétra-ester, de 39% de monoester et 61% de di , tri-, et tétra-ester, et le mono-laurate de sucrose ; les produits vendus sous la dénomination Ryoto Sugar Esters par exemple référencés B370 et correspondant au béhénate de saccharose formé de 20% de monoester et 80% de di-triester-polyester ; le mono-di-palmito-stéarate de sucrose commercialisé par la société Goldschmidt sous la dénomination Tegosoft PSE.

En ce qui concerne les ester et éthers cycliques, on peut citer par exemple la γ-butyrolactone, le diméthyl isosorbide (dénomination CTFA), ou le diisopropyl isosorbide (dénomination CTFA).

Concernant les huiles silicones, on utilise en tant que liquide inerte des composés qui sont plus particulièrement liquides et non volatiles, de préférence de viscosité inférieure ou égale à 10 000 mPa.s à 25°C, la viscosité des silicones étant mesurée selon la norme ASTM 445 Appendice C.
Les huiles de silicone sont définies plus en détail dans l'ouvrage de Walter NOLL "Chemistry and Technology of Silicones" (1968) - Academic Press.
Toutefois, parmi les huiles de silicone convenables, on peut citer notamment les huiles de silicones vendues sous les dénominations DC-200 fluid-5 mPa.s, DC-200 fluid-20 mPa.s, DC-200 fluid-350 mPa.s, DC-200 fluid- 1000 mPa.s, DC-200 fluid-10 000 mPa.s par la société Dow Corning.

L'huile de paraffine est un exemple d'huile minérale susceptible d'être utilisée comme liquide inerte de la composition de décoloration anhydre.

En ce qui concerne les huiles végétales, on peut citer tout particulièrement l'huile d'avocat, l'huile d'olive ou la cire liquide de jojoba.

### Additifs

### Polymères amphiphiles :

La pâte de décoloration anhydre peut de plus comprendre les additifs usuels tels que les polymères amphiphiles comportant au moins une chaîne hydrophobe. Plus spécialement ces polymères amphiphiles, s'ils sont présents, sont de type non ionique, anionique, cationique ou amphotère. De préférence ils sont de nature non ionique, anionique ou cationique.

Notons que le(s) polymère(s) amphiphile(s) présent(s) dans la composition de décoloration anhydre et celui présent dans la composition oxydante et qui sera détaillé plus loin, sont de préférence différents.

Plus particulièrement, lesdits polymères amphiphiles comprennent, en tant que chaîne hydrophobe, une chaîne hydrocarbonée, saturée ou non, aromatique ou non, linéaire ou ramifiée, en C₈-C₃₀, comprenant éventuellement un ou plusieurs motifs oxyalkylénés (oxyéthylénés et/ou oxypropylénés).

Parmi les polymères amphiphiles cationique comportant une chaîne hydrophobe on peut trouver des polyuréthannes cationiques ou des copolymères cationiques comprenant des motifs vinyllactame et en particulier vinylpyrrolidone.
Encore plus préférentiellement les polymères amphiphiles comportant une chaîne hydrophobe seront de nature nonionique ou anionique.

A titre d'exemples de polymères amphiphiles non ioniques à chaîne hydrophobe, on peut citer entre autres :
**(1)** les celluloses modifiées par des groupements comportant au moins une chaîne hydrocarbonée, saturée ou non, linéaire ou ramifiée, en C₆-C₃₀, comme les hydroxyéthylcelluloses modifiées par des groupements comportant au moins une chaîne hydrophobe telle que définie auparavant, comme notamment Natrosol Plus Grade 330 CS (alkyles en C₁₆ - commercialisé par la société Aqualon) ; Bermocoll EHM 100 (commercialisé par la société Berol Nobel), Amercell Polymer HM-1500 (hydroxyéthylcellulose modifiée par un groupement polyéthylène glycol (15) éther de nonylphénol - commercialisé par la société Amerchol).
**(2)** les hydroxypropylguars modifiés par des groupements comportant au moins une chaîne hydrophobe telle que définie, par exemple Jaguar XC-95/3 (chaîne alkyle en C₁₄ - commercialisé par la société Rhodia Chimie) ; Esaflor HM 22 (chaîne alkyle en C₂₂ - commercialisé par la société Lamberti) ; RE210-18 (chaîne alkyle en C₁₄) et RE205-1 (chaîne alkyle en C₂₀) commercialisés par la société Rhodia Chimie.
**(3)** les copolymères de vinylpyrrolidone et de monomères hydrophobes à chaîne hydrophobe telle que définie auparavant comme par exemple Antaron ou Ganex V216 (copolymères vinylpyrrolidone / hexadécène) ; Antaron ou Ganex V220 (copolymères vinylpyrrolidone / eicosène), commercialisés par la société I.S.P.
**(4)** les copolymères de (méth)acrylates d'alkyles en C₁-C₆ et de monomères amphiphiles comportant une chaîne hydrophobe.
**(5)** les copolymères de (méth)acrylates hydrophiles et de monomères hydrophobes comportant au moins une chaîne hydrophobe, tels que par exemple le copolymère méthacrylate de polyéthylèneglycol / méthacrylate de lauryle.
**(6)** les polymères à squelette aminoplaste éther possédant au moins une chaîne grasse, tels que les composés Pure Thix commercialisés par la société Süd-Chemie.
**(7)** les polyéthers polyuréthannes, linéaires (structure à blocs), greffés ou en étoile, comportant dans leur chaîne, au moins une séquence hydrophile, généralement polyoxyéthylénée et pouvant comprendre entre 50 et 1000 motifs oxyéthylène environ, et au moins une séquence hydrophobe, qui peut comprendre des groupements aliphatiques seuls, éventuellement combinés à des enchaînements cycloaliphatiques et/ou aromatiques. De préférence, les polyéthers polyuréthanes comportent au moins deux chaînes hydrophobes hydrocarbonées en C₆-C₃₀, séparées par une séquence hydrophile ; les chaînes hydrophobes pouvant être des chaînes pendantes ou des chaînes à l'une ou plusieurs des extrémités de la ou des séquences hydrophiles.
Les polyéthers polyuréthannes comportent une liaison uréthanne entre les séquences hydrophiles, d'où l'origine du nom. Par extension figurent aussi parmi les polyéthers polyuréthannes dont les séquences hydrophiles sont liées aux séquences lipophiles par d'autres liaisons chimiques.
Les polyéthers polyuréthannes utilisables selon l'invention sont en particulier ceux décrits dans l'article de G. Fonnum, J. Bakke et Fk. Hansen - Colloid Polym. Sci 271, 380.389 (1993). A titre d'exemples de polyéthers polyuréthannes, on peut citer Nuvis FX 1100 (désignation I.N.C.I. européenne et américaine "Steareth-100/PEG-136/H.M.D.I. Copolymer" commercialisé par la société Servo Delden) ; Rhéolate 205, 208 , 204 ou 212 (commercialisés par la société Rheox) ; Elfacos T210 (chaîne alkyle en C₁₂₋ C₁₄) Elfacos T212 (chaîne alkyle en C₁₈) comemrcialisés par la société Akzo.

Les polymères amphiphiles anioniques à chaîne hydrophobe, susceptibles d'être mis en oeuvre comportent au moins à titre de chaîne hydrophobe, une chaîne hydrocarbonée, saturée ou non, aromatique ou non, linéaire ou ramifiée, en C₈-C₃₀.

Plus particulièrement les polymères amphiphiles anioniques comportant au moins une chaîne hydrophobe utilisables dans le cadre de la présente invention, réticulés ou non, comprennent au moins un motif hydrophile dérivé d'un ou de plusieurs monomères à insaturation éthylénique portant une fonction acide carboxylique libre, ou une fonction sulfonique libre, ou partiellement ou totalement neutralisée, et au moins un motif hydrophobe dérivé d'un ou de plusieurs monomères à insaturation éthylénique portant une chaîne latérale hydrophobe, et éventuellement au moins un motif de réticulation dérivés d'un ou plusieurs monomères polyinsaturés.

A titre de monomère à insaturation éthylénique portant une fonction acide carboxylique, on peut citer l'acide éthacrylique, l'acide méthacrylique et l'acide acrylique, ou leurs mélanges ; les deux derniers étant préférés.
A titre d'exemples de monomères à insaturation éthylénique portant une chaîne latérale hydrophobe peuvent être cités les esters d'acides carboxyliques insaturés, comme notamment les acides éthacrylique, méthacrylique, acrylique, et d'alcools saturés, linéaires ou ramifiés, en C₁₀-C₃₀, plus particulièrement en C₁₂-C₂₂. Ils peuvent aussi être des éthers allyliques d'alcools saturés ou non, aromatiques ou non, ramifiés ou non, en C₆-C₃₀, éventuellement oxyalkyléné (de préférence oxyéthyléné), plus particulièrement de formule CH₂=CR'CH₂OBₙR, avec R' représentant H ou CH₃, B représentant le radical éthylèneoxy, n est un entier compris entre 0 et 100, R représente un radical hydrocarboné choisi parmi les radicaux alkyle, arylalkyle, aryle, alkylaryle, cycloalkyle, comportant de 8 à 30 atomes de carbone. Un motif plus particulièrement préféré est tel que R' représente l'hydrogène, n est égal à 10, et R représente un radical stéaryle (C₁₈). Concernant le monomère réticulant, celui-ci comprend au moins deux doubles liaisons polymérisables non conjuguées l'une avec l'autre. On peut citer à titre d'exemples le phtalate de diallyle, le (méth)acrylate d'allyle, le divinylbenzène, le diméthacrylate de (poly)éthylèneglycol, le méthylène-bis-acrylamide, le polyallylsucrose ou le polyallyl-pentaérythritol.
Des polymères amphiphiles anioniques du type décrit ci-dessus sont décrits et préparés, par exemple dans les brevets US 3915921 et US 4509949 (copolymères d'acide (méth)acrylique et de (méth)acrylates d'alkyles en C₁₀-C₃₀) ou dans le brevet EP 216479 (copolymères d'acide (méth)acrylique et d'éthers allyliques d'alcools gras).

On peut citer à titre d'exemples de polymères préférés, Carbopol ETD-2020 (copolymère acide acrylique/méthacrylate d'alkyle en C₁₀-C₃₀, réticulé - commercialisé par la société Goodrich) ; Carbopol 1382, Pemulen TR1, Pemulen TR2 (copolymères acide acrylique/acrylate d'alkyle en C₁₀₋₃₀, réticulés - commercialisés par la société Goodrich).

Les polymères amphiphiles comportant comme motifs hydrophiles au moins un monomère à insaturation éthylénique à groupement sulfonique, sous forme libre ou partiellement ou totalement neutralisée et au moins une partie hydrophobe sont par exemple décrits dans FR 0016954 et FR 0100328 dont le contenu fait partie intégrante de la présente invention.
On peut citer plus particulièrement parmi eux le copolymère acide acrylamido-2-méthyl-2-propane-sulfonique(AMPS)/n-dodécylacrylamide neutralisé par la soude, le copolymère réticulé par du méthylène-bis-acrylamide constitué de 75% en poids de motifs AMPS neutralisés par NH₃ et de 25% en poids de motifs acrylate de Genapol T-250, le copolymère réticulé par du méthacrylate d'allyle constitué de 90% en poids de motifs AMPS neutralisés par NH₃ et de 10% en poids de motifs méthacrylate de Genapol T-250, ou le copolymère réticulé par du méthacrylate d'allyle constitué de 80% en poids de motifs AMPS neutralisés par NH₃ et de 20% en poids de motifs méthacrylate de Genapol T-250.

Si ces polymères amphiphiles sont présents, leur teneur représente de 0,01 à 30 % en poids de la composition de décoloration prête à l'emploi.

*Polymères hydrosolubles épaississants* dépourvus de chaîne hydrophobe :
La composition de décoloration anhydre peut de plus comprendre au moins un polymère hydrosoluble épaississant dépourvu de chaîne hydrophobe.

Les polymères convenables peuvent être d'origine naturelle, ou être des polymères synthétiques, et sont avantageusement choisis parmi ceux utilisés classiquement dans le domaine cosmétique. En outre, ces polymères ne présentent pas de chaîne hydrophobe, c'est-à-dire une chaîne hydrocarbonée, saturée ou non, aromatique ou non, linéaire ou ramifiée, en C₈-C₃₀, comprenant éventuellement un ou plusieurs motifs oxyalkylénés (oxyéthylénés et/ou oxypropylénés). Les polymères hydrosolubles épaississants sont donc différents des polymères amphiphiles qui viennent d'être décrits.

Comme exemples de polymères synthétiques, on peut citer, la polyvinylpyrrolidone, l'acide polyacrylique, le polyacrylamide, l'acide poly-2-acrylamidopropanesulfonique non réticulé (Simugel EG de la société SEPPIC), l'acide poly-2-acrylamido-2-méthylpropane sulfonique réticulé, libre ou partiellement neutralisé par l'ammoniaque (Hostacerin AMPS de Clariant), des mélanges d'acide poly-2-acrylamido-2-méthylpropane sulfonique non réticulé avec des éthers d'hydroxyalkylcellulose ou avec des poly(oxyde d'éthylène) tel qu'ils sont décrits dans le brevet US 4540510 ; des mélanges d'acide poly(méth)acrylamido-alkyl(C₁-C₄)-sulfonique, de préférence réticulé, avec un copolymère réticulé de l'anhydride maléique et d'un alkyl(C₁-C₅)vinyléther (Hostacerin AMPS / Stabileze QM de la société ISF).

Les polymères épaississants d'origine naturelle utilisables selon la présente invention, sont de préférence des polymères comportant au moins un motif sucre, comme les gommes de guar non ioniques, modifiées ou non par des groupements hydroxyalkyle en C₁-C₆ ; les gommes de biopolysaccharides d'origine microbienne telles que les gommes de Scléroglucane ou de Xanthane ; les gommes issues d'exudats végétaux telles que les gommes Arabique, Ghatti, Karaya, Tragacanthe, Carraghénanne, Agar et Caroube ; les pectines ; les alginates ; les amidons ; les hydroxyalkyl(C₁-C₆)celluloses et carboxyalkyl(C₁-C₆)celluloses.

Ici, "motif sucre" désigne une portion monosaccharidique (i.e. monosaccharide ou oside ou sucre simple), une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides]. Les unités saccharidiques peuvent être en outre substituées par des radicaux alkyle, ou hydroxyalkyle, ou alcoxy, ou acyloxy, ou carboxyle, les radicaux alkyle comportant de 1 à 4 atomes de carbone.

A titre d'exemples de gommes de guar non ioniques non modifiées, on peut citer entre autres Guargel D/15 (Goodrich) ; Vidogum GH 175 (Unipectine), Meypro-Guar 50 et Jaguar C (Meyhall/Rhodia Chimie) ; et à titre de gommes de guar non ioniques modifiées, Jaguar HP8, HP60, HP120, DC 293, HP 105 (Meyhall/Rhodia Chimie) ; Galactasol 4H4FD2 (Aqualon).

Les gommes de biopolysaccharides d'origine microbienne, végétale sont bien connues de l'homme de l'art et décrites notamment dans l'ouvrage de Robert L. Davidson intitulé "Handbook of Water soluble gums and resins" édité chez Me Graw Hill Book Company (1980).
Parmi ces gommes, citons les scléroglucanes comme notamment Actigum CS de Sanofi Bio Industries ; Amigel de Alban Muller International, ainsi que les scléroglucanes traités au glyoxal décrits dans FR 2633940) ; les gommes xanthanes comme Keltrol, Keltrol T, Keltrol Tf, Keltrol Bt, Keltrol Rd, Keltrol Cg (Nutrasweet Kelco), Rhodicare S, Rhodicare H (Rhodia Chimie) ; les dérivés d'amidon comme Primogel (Avebe) ; les hydroxyéthylcelluloses telles que Cellosize QP3L, QP4400H, QP30000H, HEC30000A, Polymer PCG10 (Amerchol), Natrosol 250HHR, 250MR, 250M, 250HHXR, 250HHX, 250HR, HX (Hercules), Tylose H1000 (Hoechst) ; les hydroxypropylcelluloses comme Klucel EF, H, LHF, MF, G (Aqualon) ; les carboxyméthylcelluloses comme Blanose 7M8/SF, raffinée 7M, 7LF, 7MF, 9M31F, 12M31XP, 12M31P, 9M31XF, 7H, 7M31, 7H3SXF (Aqualon), Aquasorb A500 (Hercules), Ambergum 1221 (Hercules), Cellogen HP810A, HP6HS9 (Montello), Primellose (Avebe).

Les polymères épaississants hydrosolubles dépourvus de chaîne hydrophobe, s'ils sont présents, représentent de 0,01 à 30 % en poids de la composition de décoloration anhydre.

### Tensioactifs :

La composition de décoloration anhydre peut de plus comprendre un ou plusieurs tensioactifs, choisis parmi les tensioactifs anioniques, non ioniques, cationiques ou amphotères.

A titre d'exemples non limitatifs de tensioactifs anioniques utilisables, seuls ou mélanges, dans le cadre de la présente invention, on peut citer notamment les sels (en particulier sels alcalins, notamment de sodium, magnésium, sels d'ammonium, d'amines, d'aminoalcools) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglycoside citrates, tartrates et sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, et palmitique, des acides d'huile de coprah ou d'huile de coprah hydrogénée, et notamment de préférence les sels de sodium, calcium ou magnésium de l'acide stéarique; les acyl-lactylates dont le radical acyle comporte ô à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Sans vouloir s'y limiter, les tensioactifs non ioniques peuvent être choisis, seuls ou en mélange, parmi les alcools, les alpha-diols, les alkylphénols polyéthoxylés et/ou polypropoxylés, ayant une chaîne comportant par exemple 8 à 22 atomes de carbone, le nombre de groupements oxyde d'éthylène et/ou oxyde de propylène pouvant aller notamment de 1 à 50. On peut également citer les copolymères d'oxyde d'éthylène et de propylène, les condensats d'oxyde d'éthylène et de propylène sur des alcools gras (comprenant par exemple 8 à 22 atomes de carbone) ; les amides gras (notamment en C₈-C₂₂) polyéthoxylés ayant de préférence de 2 à 30 moles d'oxyde d'éthylène, les alcools gras (notamment en C₈-C₂₂) mono- ou poly-glycérolés comportant en moyenne 1 à 30 groupements glycérol et les amides gras (notamment en C₈-C₂₂) polyglycérolés comportant en moyenne 1 à 5 groupements glycérol et en particulier 1,5 à 4 ; les esters d'acides gras (notamment en C₈-C₂₂) du sorbitan oxyéthylénés ayant par exemple de 2 à 30 moles d'oxyde d'éthylène ; les esters d'acides gras (notamment en C₈-C₂₂) du sucrose, les esters d'acides gras (notamment en C₈-C₂₂) du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

A titre d'illustration, les agents tensioactifs amphotères ou zwitterioniques peuvent être notamment des dérivés d'amines secondaires ou tertiaires aliphatiques, dans lesquels le radical aliphatique est une chaîne linéaire ou ramifiée, en C₈-C₁₈, et contenant au moins un groupe anionique du type carboxylate, sulfonate, sulfate, phosphate ou phosphonate ; des alkyl(C₈-C₂₀)bétaïnes, les alkyl(C₈-C₂₀)sulfobétaïnes, les alkyl(C₈-C₂₀) amidoalkyl (C₁-C₆)bétaïnes ou les alkyl(C₈-C₂₀)amidoalkyl(C₁-C₆)sulfobétaïnes. Conviennent aussi les ampho- carboxyglycinates les amphocarboxypropionates, classés dans le dictionnaire CTFA, 5ème édition, 1993, notamment sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauro-amphodipropionate, Disodium Caprylampho-dipropionate, Disodium Capryloampho-dipropionate, Lauroamphodipropionic acid, Cocoamphodipropionic acid. A titre d'exemples on peut citer le Cocoamphodiacétate (Miranol^{®} C2M concentré de Rhodia Chimie).

Parmi les tensioactifs cationiques on peut citer à titre purement indicatif, les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

De préférence, s'ils sont présents, les tensioactifs sont choisis parmi les composés anioniques et/ou non ioniques.

Au cas où un ou plusieurs tensioactifs seraient présents dans la composition de décoloration anhydre, leur teneur est telle que la teneur totale en tensioactif dans la composition prête à l'emploi représente de 0,05 à 30 %, et de préférence 0,1 à 20 % en poids.

### Polymère substantif cationique ou amphotère :

Selon une variante de l'invention, la composition de décoloration comprend au moins un polymère substantif cationique ou amphotère. Ce type de polymères permet d'améliorer les propriétés cosmétiques des fibres (effet conditionneur).
Rappelons que, au sens de la présente invention, l'expression "polymère cationique" désigne tout polymère contenant des groupements cationiques et/ou des groupements ionisables en groupements cationiques.

Ces polymères cationiques ou amphotères convenables sont de manière avantageuse, choisis parmi ceux déjà connus en soi comme améliorant les propriétés cosmétiques des cheveux, à savoir notamment ceux décrits dans les brevets et demandes de brevet EP 337 354, FR 2 270 846, FR 2 383 660, FR 2 598 611, FR 2 470 596, FR 2 519 863, FR 2 788 974, FR 2 788 976 pour avoir une liste de ces composés.

Cependant, à titre d'exemples plus précis de polymères cationiques, on peut notamment citer les polymères cationiques comprenant au moins des groupements amine primaire, secondaire, tertiaire et/ou quaternaire pouvant, soit faire partie de la chaîne principale polymère, soit être portés par un substituant latéral directement relié à celle-ci.

A titre d'exemples plus précis de ces polymères, on peut citer :
**(1)** les copolymères d'acrylamide et de diméthyl-amino-éthyl méthacrylate quaternisé au sulfate de diméthyle ou avec un halogénure de diméthyle (Hercofloc de Hercules) ; les copolymères d'acrylamide et de chlorure de méthacryloyloxy-éthyl-triméthylammonium (Bina Quat P 100 de Ciba Geigy) ; le copolymère d'acrylamide et de méthosulfate de méthacryloyloxy-éthyl-triméthylammonium (Reten de Hercules) ; les copolymères vinylpyrrolidone / acrylate ou méthacrylate de dialkylaminoalkyle quaternisés ou non (gamme Gafquat d'ISP ; Copolymer 845, 958 et 937 de Gaf Corporation (ISP ?)) ; les terpolymères méthacrylate de diméthyl amino éthyle/ vinylcaprolactame/ vinylpyrrolidone (Gaffix VC 713 d'ISP) ; les copolymères vinylpyrrolidone / méthacrylamidopropyl dimethylamine (Styleze CC 10 d'ISP) ; les copolymères vinylpyrrolidone / méthacrylamide de diméthyl-amino-propyle quaternisés (Gafquat HS 100 d'ISP).
**(2)** Les dérivés d'éthers de cellulose comportant des groupements ammonium quaternaire tels que décrits dans FR 1 492 597. Ces polymères sont également définis dans le dictionnaire CTFA comme des ammonium quaternaires d'hydroxyéthylcellulose ayant réagi avec un époxyde substitué par un groupement triméthylammonium.
**(3)** Les dérivés de cellulose cationiques tels que les copolymères de cellulose ou les dérivés de cellulose greffés avec un monomère hydrosoluble d'ammonium quaternaire, et décrits notamment dans US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylamidopropyl-triméthylammonium, de diméthyl-diallylammonium.
**(4)** Les polysaccharides cationiques décrits plus particulièrement dans les brevets US 3 589 578, US 4 031 307, tels que les gommes de guar contenant des groupements cationiques trialkylammonium. On utilise par exemple des gommes de guar modifiées par un sel, comme le chlorure notamment, de 2,3-époxypropyl triméthylammonium.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans FR 2 162 025, FR 2 280 361.
**(6)** Les polyaminoamides solubles dans l'eau préparés en particulier par polycondensation d'un composé acide avec une polyamine, éventuellement réticulés, éventuellement alcoylés ou s'ils comportent une ou plusieurs fonctions amines tertiaires, quaternisées. Ces polymères sont notamment décrits dans FR 2 252 840 et FR 2 368 508.
**(7)** Les dérivés de polyaminoamides résultant de la condensation de polyalkylènes polyamines avec des acides polycarboxyliques suivie d'une alcoylation par des agents bifonctionnels. On peut citer par exemple les polymères acide adipique-dialkylamino hydroxyalkyldialkylène triamine dans lesquels le radical alkyle est en C1-C4. De tels polymères sont notamment décrits dans FR 1 583 363.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés en C3-C8, puis avec l'épichlorhydrine. De tels polymères sont notamment décrits dans US 3 227 615, US 2 961 347.
**(9)** Les cyclopolymères d'alkyl diallyl amine ou de dialkyl diallyl ammonium, sous forme d'homopolymère ou de copolymère, tels que décrits dans FR 2 080 759 et dans son certificat d'addition n°2 190 406.
**(10)** les polymères de diammonium quaternaire tels que décrits dans FR 2 320 330, FR 2 270 846, FR 2 316 271, FR 2 336 434, FR 2 413 907, US 2 273 780, US 2 375 853, US 2 388 614, US 2 454 547, US 3 206 462, US 2 261 002, US 2 271 378, US 3 874 870, US 4 001 432, US 3 929 990, US 3 966 904, US 4 005 193, US 4 025 617, US 4 025 627, US 4 025 653, US 4 026 945, US 4 027 020.
   On peut utiliser plus particulièrement les polymères qui sont constitués de motifs récurrents répondant à la formule suivante: 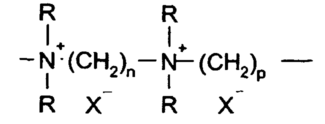 dans laquelle les radicaux R, identiques ou non, désignent un radical alkyle ou hydroxyalkyle en C1-C4, n et p sont des nombres entiers variant de 2 à 20 et, X⁻ est un anion dérivé d'un acide minéral ou organique.
**(11)** Les polymères de poly(ammonium quaternaire) constitués de motifs récurrents de formule : 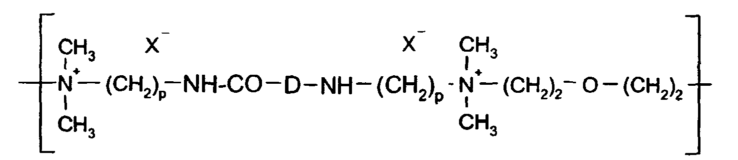 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion. De tels polymères peuvent être préparés selon les procédés décrits dans US 4 157 388, US 4 702 906, US 4 719 282, EP 122 324.
**(12)** Les polymères quaternaires de vinylpyrrolidone et de vinylimidazole.
**(13)** Les polyamines du type Polyethyleneglycol (15) Tallow Polyamine(dénomination du dictionnaire CTFA).
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale (Salcare^{®} SC 92 de Allied Colloids). On peut également utiliser un homopolymère réticulé du chlorure de méthacryloyloxyéthyl triméthylammonium contenant environ 50 % en poids de l'homopolymère dans de l'huile minérale ou dans un ester liquide (Salcare^{®} SC 95, SC 96 de Allied Colloids).

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

En ce qui concerne les polymères amphotères, on peut utiliser des polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a réagi avec une polyamine comportant un ou plusieurs groupements amine primaire ou secondaire.

Les polymères amphotères répondant à la définition donnée ci-dessus plus particulièrement préférés sont choisis parmi les polymères suivants :
**(1)** Les polymères résultant de la copolymérisation d'un monomère dérivé d'un composé vinylique portant un groupement carboxylique tel que plus particulièrement l'acide (méth)acrylique, l'acide maléique, l'acide alpha-chloracrylique, ou encore un sel de dialkyldiallylammonium tel que le chlorure de diméthyldiallylammonium, et d'un monomère basique dérivé d'un composé vinylique substitué contenant au moins un atome basique tel que plus particulièrement les dialkyl-amino-alkyl-méthacrylate et acrylate, les dialkyl-amino-alkyl-méthacrylamide et acrylamide, comme décrits dans US 3 836 537. On peut citer le copolymère acrylate de sodium / chlorure d'acrylamidopropyl trimethyl ammonium (Polyquart KE 3033 de Henkel), le copolymère acide acrylique/ chlorure de diméthyldiallylammonium (Merquat 280, 295, Plus 3330, de Calgon).
**(2)** Les polymères comportant des motifs dérivant a) d'au moins un monomère choisi parmi les (méth)acrylamides N-substitués par un radical alkyle, notamment en C₂-C₁₂ (par exemple éthyl, tertiobutyl, tertiooctyl, octyl, décyl, le dodécyl), b) d'au moins un monomère acide contenant un ou plusieurs groupements carboxyliques réactifs (par exemple acides (méth)acrylique, crotonique, itaconique, et les monoesters des acides ou anhydrides maléique, fumarique), et c) d'au moins un monomère basique tel que des esters à substituant amine primaire, secondaire, tertiaire et quaternaire des acides (méth)acrylique, fumarique, maléique, et le produit de quaternisation du méthacrylate de diméthyl-amino-éthyle avec le sulfate de diméthyle ou diéthyle (par exemple les méthacrylates d'aminoéthyle, de butylaminoéthyle, deN,N'-diméthyl-aminoéthyle, de N-tertio-butyl-aminoéthyle.)
   On utilise particulièrement les copolymères Octylacrylamide / acrylate / butylaminoéthyl-méthacrylate (Amphomer ou Lovocryl 47 par la société National Starch).
**(3)** Les polyaminoamides réticulés et partiellement ou totalement alcoylés, dérivant de polyaminoamides de formule générale -[CO-R-CO-Z]- dans laquelle R est un radical divalent dérivé d'un acide dicarboxylique saturé ou non (par exemple les acides adipique, triméthyl-2,2,4-adipique et triméthyl-2,4,4-adipique, téréphtalique, itaconique), d'un acide monocarboxylique insaturé (comme l'acide (méth)acrylique), d'un ester d'alcool en C₁-C₆ des acides précités ou d'un radical dérivant de l'addition de l'un de ces acides avec une amine bis-primaire ou -secondaire, et Z désigne un radical d'une polyalkylène-polyamine bis-primaire, mono ou bis-secondaire. De préférence, Z représente entre 60 et 100 moles %, le radical -NH-[(CH₂)ₓ-NH]ₚ- avec x=2 et p=2 ou 3, ou x=3 et p=2 ; ce radical dérivant de la diéthylène triamine, de la triéthylène tétraamine ou de la dipropylène triamine; entre 0 et 40 moles % le radical ci-dessus, dans lequel x=2 et p=1 et qui dérive de l'éthylènediamine, ou le radical dérivant de la pipérazine -N[CH₂CH₂]₂N- ; entre 0 et 20 moles %, le radical -NH-(CH₂)₆-NH- dérivant de l'hexaméthylènediamine. L'agent réticulant de ces polymères est un agent bifonctionnel choisi parmi les épihalohydrines, les diépoxydes, les dianhydrides, les dérivés bis insaturés, et alcoylés par action d'acide acrylique, d'acide chloracétique ou d'une alcane sultone (comme la propane ou la butane sultone) ou de leurs sels de métaux alcalins.
**(4)** Les polymères comportant au moins des motifs zwittérioniques, comme par exemple le copolymère de méthacrylate de butyle / méthacrylate de diméthylcarboxy-méthyl-ammonio-éthyle (Diaformer Z301 de Sandoz).
**(5)** Les polymères dérivés du chitosane comportant des motifs monomères répondant aux formules (I), (II), (III) suivantes : 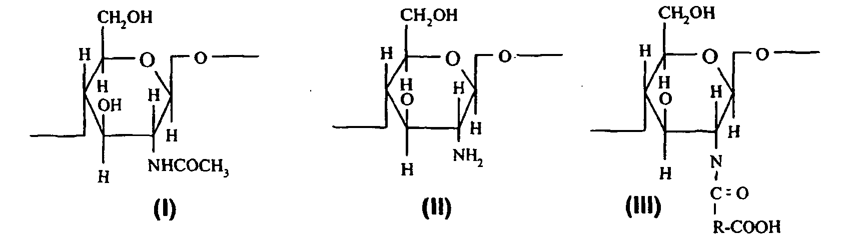 avec (I) représentant de 0 à 30%, (II) de 5 à 50% et (III) de 30 à 90% dans lequel R représente un radical de formule : 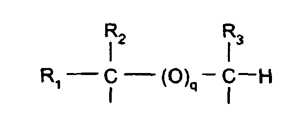 dans laquelle q désigne zéro ou 1 ; et si q=0, Ri, identiques ou non, représentent un hydrogène, un groupement méthyle, hydroxyle, acétoxy, amino, mono- ou di-alkylamine éventuellement interrompus par un ou plusieurs atomes d'azote et/ou éventuellement substitués par un ou plusieurs groupes amine, hydroxyle, carboxyle, alkylthio éventuellement porteur d'un groupe amino, sulfonique ; ou si q=1, Ri, identiques ou non, représentent un hydrogène, ainsi que les sels formés par ces composés avec des bases ou des acides.
**(6)** Les polymères dérivés de la N-carboxyalkylation du chitosane comme le N-carboxyméthyl chitosane ou le N-carboxybutyl chitosane (Evalsan de Jan Dekker).
**(7)** Les polymères tels que décrits dans FR 1 400 366 : 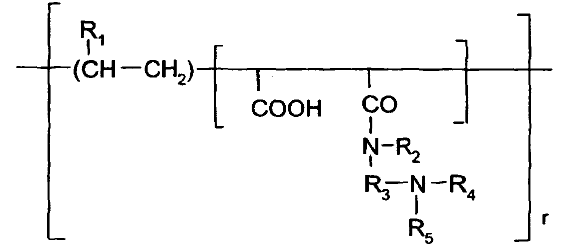 dans laquelle R₁ est un hydrogène, CH₃O-, CH₃CH₂O-, phényle, R₂, R₅, identiques ou non, représentent un hydrogène, un radical alkyle (méthyle, éthyle), R₄ représente un radical alkyle (méthyle, éthyle) ou un radical de formule -R₃-N(R₅)₂, R₃ représentant -(CH₂)₂-, -(CH₂)₃-, -CH₂-CH(CH₃)-, ainsi que les homologues supérieurs de ces radicaux et contenant jusqu'à 6 atomes de carbone, r est tel que le poids moléculaire est compris entre 500 et 6000000 et de préférence entre 1000 et 1000000.
**(8)** Les polymères amphotères du type -D-X-D-X- choisis parmi:
   a) Les polymères obtenus par action de l'acide chloracétique ou le chloracétate de sodium sur les composés comportant au moins un motif de formule -D-X-D-X-D- où D désigne un radical -N[CH₂CH₂]₂N- (pipérazinyle) et X désigne le symbole E ou E', E ou E' identiques ou différents désignent un radical bivalent qui est un radical alkylène à chaîne droite ou ramifiée comportant jusqu'à 7 atomes de carbone dans la chaîne principale substituée ou non par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule -D-X-D-X- où D désigne un radical -N[CH₂CH₂]₂N-(pipérazinyle) et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
**(9)** Les copolymères alkyl(C₁-C₅)-vinyléther / anhydride maléique modifié partiellement par semi-amidification avec une N,N-dialkylaminoalkylamine telle que la N,N-diméthyl-amino-propylamine ou par semi-estérification avec une N,N-dialcanolamine. Ces copolymères peuvent également comporter d'autres comonomères vinyliques tels que le vinylcaprolactame.

Parmi tous les polymères cationiques ou amphotères utilisables selon la présente invention, on préfère notamment :
(i) parmi les cationiques :
   - l'homopolymère de chlorure de diméthyldiallylammonium (Merquat 100 de NALCO);
   - les copolymères de chlorure de diméthyldiallylammonium et d'acrylamide (Merquat 2200 de NALCO);
   - les polymères de type poly(ammonium quaternaire) préparés et décrits dans FR 2 270 846, constitués de motifs récurrents de formules (W) et (U) suivantes : 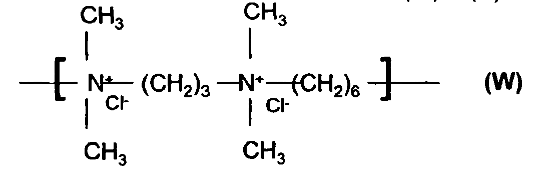 et notamment ceux dont le poids moléculaire, déterminé par chromatographie par perméation de gel, est compris entre 9500 et 9900; 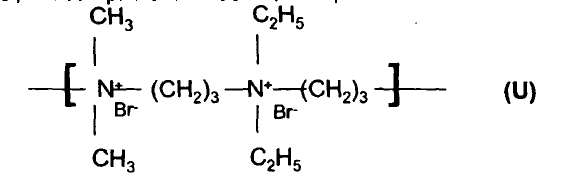 et notamment ceux dont le poids moléculaire, déterminé par chromatographie par perméation de gel, est d'environ 1200;
   - les polymères de type poly(ammonium quaternaire) de la famille (11) avec X⁻ désignant le Chlore, et notamment ceux dont la masse moléculaire moyenne en poids est inférieure à 100 000, de préférence inférieure ou égale à 50 000;
(ii) parmi les polymères amphotères :
   - le copolymère chlorure de diméthyldiallylammonium/acide acrylique (80/20) (Merquat 280 DRY de Calgon- dénomination CTFA : Polyquaternium 22) ;
   - le copolymère chlorure de diméthyldiallylammonium/acide acrylique (95/5) (Morquat 295 DRY de Calgon) ;
   - le copolymère de chlorure de méthacrylamidopropyltrimonium, d'acide acrylique et d'acrylate d'éthyle (Merquat 2001 de Calgon - dénomination CTFA : Polyquaternium 47) ;
   - le terpolymère acrylamide/chlorure de diméthyldiallylammonium/acide acrylique (Merquat Plus 3330 DRY de Calgon -dénomination CTFA : Polyquaternium 39).

### Autres additifs

La composition de décoloration anhydre peut de même comprendre au moins une charge minérale, comme les argiles, les silices telles les silices pyrogénées à caractère hydrophile ou hydrophobe.
Elle peut de plus comprendre au moins un liant tel que la vinylpyrrolidone, au moins un lubrifiant comme les stéarates de polyol ou les stéarates de métaux alcalins ou alcalino-terreux, ainsi que des agents de contrôle du dégagement d'oxygène tels que le carbonate ou l'oxyde de magnésium.
La composition de décoloration anhydre peut comprendre le cas échéant un ou plusieurs agents choisis parmi les colorants, les agents matifiants comme les oxydes de titane, les agents séquestrants, les vitamines ou provitamines, les filtres solaires, les silicones, les parfums.

Bien entendu, l'homme de l'art veillera à choisir le ou les éventuels composés complémentaires mentionnés ci-avant, de manière telle que les propriétés avantageuses attachées intrinsèquement à la composition de décoloration et/ou à la composition de décoloration prête à l'emploi ne soient pas, ou substantiellement pas, altérées par la ou les adjonctions envisagées.

La composition de décoloration anhydre, sous forme de pâte, peut être préparée de manière classique, en dispersant, sous agitation mécanique, l'ensemble des composés pulvérulents dans le liquide inerte, dans lequel on a préalablement dispersé ou mélangé les autres composés liquides de la composition de décoloration.
On peut également préparer la pâte par extrusion, en introduisant les phases liquides et solides de la composition dans l'extrudeur, puis en les mélangeant à une température inférieure à 25°C à l'aide d'un système bi-vis co-rotatives composé d'éléments de transport et de malaxage.

La composition oxydante va maintenant être décrite.

### Composition oxydante

Cette dernière est une émulsion huile dans eau de peroxyde d'hydrogène, comprenant au moins un tensioactif non ionique et/ou anionique et au moins un copolymère comprenant au moins un motif provenant d'un acide (méth)acrylique salifié ou non et au moins un motif provenant d'un d'un ester d'acide (méth)acrylique avec un alcool gras en C₈-C₃₀, saturé ou insaturé, linéaire ou ramifié, éthoxylé.

### Tensioactifs

En ce qui concerne les tensioactifs non ioniques et anioniques, on pourra se référer aux listes détaillées précédemment dans le cadre de la description des éléments constitutifs de la composition de décoloration anhydre.

De préférence, on met en oeuvre au moins un tensioactif choisi parmi les tensioactifs non ioniques polyalcoxylés ou polyglycérolés, de préférence polyéthoxylés. A titre d'exemple préféré, on peut citer les alcools ayant une chaîne en C₈-C₂₂ atomes de carbone, le nombre de groupements oxyde d'éthylène étant compris entre 1 et 50.

Il est à noter que la teneur en tensioactif(s) présent(s) dans la composition oxydante est en général comprise entre 0,05 et 30 % en poids, de préférence entre 0,1 et 20 % en poids.
Avantageusement, la composition prête à l'emploi, c'est-à-dire la composition de décoloration anhydre et la composition oxydante, présente une teneur totale en tensioactifs comprise entre 0,05 et 30 %, et de préférence entre 0,1 et 20 % en poids de la composition prête à l'emploi.

### Copolymère

Comme indiqué auparavant, le copolymère présent dans l'émulsion huile dans eau comprend au moins un motif provenant d'un acide (méth)acrylique salifié ou non et au moins un motif provenant d'un d'un ester d'acide (méth)acrylique avec un alcool gras en C₈-C₃₀, saturé ou insaturé, linéaire ou ramifié, éthoxylé.
Plus particulièrement le ou les motifs provenant d'un ester sont obtenus à partir d'un ester d'acide acrylique ou méthacrylique et d'un alcool saturé en C₁-C₆, de préférence en C₁-C4.
Le ou les motifs provenant d'acides présents dans ces copolymères, peuvent être éventuellement neutralisés, totalement ou partiellement, par un ou plusieurs agents alcalins minéraux ou organiques.

Eventuellement, le polymère peut comprendre des motifs provenant d'au moins un monomère polyéthyléniquement insaturé, comme par exemple le diallylphthalate, le divinylbenzène, le méthacrylate d'allyle, le diméthacrylate d'éthylèneglycol, notamment.

Conviennent à la réalisation de l'invention, le copolymère acide méthacrylique/acrylate d'éthyle/méthacrylate de stéaryle oxyéthyléné (55/35/10), le copolymère acide (méth)acrylique/acrylate d'éthyle/méthacrylate de béhényle oxyéthyléné 25 OE

De préférence, ledit copolymère comprend au moins un motif dérivé d'acide (méth)acrylique, au moins un motif dérivé d'acrylate d'alkyle (notamment éthyle) et au moins un motif dérivé de méthacrylate de béhényle éthoxylé, de préférence comprenant 25 moles d'oxyde d'éthylène.
Ce type de polymère est bien connu et est notamment commercialisé par Rohm & Haas sous la dénomination Aculyn 28.

Selon un mode de réalisation particulier de l'invention, la teneur en copolymère représente 0,005 à 15 % en poids de la composition prête à l'emploi, plus particulièrement de 0,05 à 7,5 % en poids de la composition prête à emploi, de préférence de 0,1 à 5 % en poids de la composition prête à l'emploi.

### Phase huile de l'émulsion

La phase huile de émulsion comprend de préférence au moins un alcool gras.

Par alcool gras selon l'invention, on entend tout alcool gras, saturé ou insaturé, linéaire ou ramifié. Parmi ces alcools gras on préfère ceux en C₁₂-C₂₂.
On peut citer plus particulièrement parmi eux, les alcools laurique, cétylique, stéarylique, oléïque, béhénique, linoléïque, undécylénique, palmitoléïque, linolénique, arachidonique, érucique, et leurs mélanges. L'alcool cétylique est plus particulièrement préféré.

Dans les émulsions oxydantes selon l'invention, la concentration en alcools gras peut varier d'environ 0,1 à 30% en poids et plus préférentiellement d'environ 0,5 à 15% en poids par rapport au poids total de l'émulsion.

### Additifs

La composition oxydante peut de même comprendre les additifs usuels dans le domaine, comme par exemple des agents séquestrants tels l'éthylènediaminetétraacétique, le pentasodium pentetate (dénomination CTFA)) ou l'acide étidronique ; des agents stabilisants l'eau oxygénée tels les sels de stannate et de pyrophosphate de métaux alcalins (comme le sodium, le potassium par exemple), ou le salicylate de sodium ; des colorants ; des parfums ; des agents anti-mousse ; des polymères substantifs cationiques ou amphotères tels que ceux décrits plus haut.

Bien entendu, l'homme de l'art veillera à choisir le ou les éventuels composés complémentaires mentionnés ci-avant, afin que les propriétés de la composition de décoloration prête à l'emploi ne soient pas, ou substantiellement pas, altérées par la ou les adjonctions envisagées.

La teneur en peroxyde d'hydrogène dans la composition oxydante est avantageusement comprise entre 1 et 12 % en titre en peroxyde d'hydrogène, plus particulièrement comprise entre 2 et 12 % en titre en peroxyde d'hydrogène, et de préférence entre 2,7 et 12 % en titre en peroxyde d'hydrogène.

La teneur en peroxyde d'hydrogène dans la composition de décoloration prête à l'emploi est, selon un mode de réalisation particulier de l'invention, comprise entre 1 et 12 % en titre en peroxyde d'hydrogène, de préférence de 2 à 9 % en titre en peroxyde d'hydrogène, et plus préférentiellement de 2 à 6 % en titre en peroxyde d'hydrogène.

Plus particulièrement, le pH de la composition oxydante est compris entre 1 et 6, et préférentiellement entre 2 et 4.
Le pH acide garantit la stabilité du peroxyde d'hydrogène dans la composition oxydante.
Il peut être obtenu à l'aide d'agents acidifiants tels que par exemple l'acide chlorhydrique, l'acide acétique, l'acide phosphorique, l'acide lactique, l'acide citrique, l'acide salicylique ou l'acide borique.
En outre, le pH peut être ajusté classiquement et si nécessaire par ajout d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

La composition oxydante est préparée en mélangeant à température ambiante le peroxyde d'hydrogène et les autres ingrédients de la phase aqueuse de l'émulsion huile dans eau puis de réaliser l'émulsion par ajout de la phase huile de l'émulsion, à une température supérieure à la température ambiante.

Le procédé de préparation de la composition de décoloration prête à l'emploi selon l'invention consiste à mélanger la composition de décoloration anhydre et la composition oxydante. Ce mélange doit se faire immédiatement avant l'application du produit sur les fibres à décolorer.

Habituellement, on mélange la composition de décoloration avec environ 0,5 à 10 équivalents en poids de la composition oxydante.

Notons que le pH de la composition prête à l'emploi selon l'invention est, avantageusement compris entre 4 et 12, plus particulièrement entre 7 et 11,5, et de préférence entre 8 et 11.

La présente invention a de même pour objet un procédé de décoloration de fibres kératiniques humaines, en particulier des cheveux, consistant à appliquer sur la zone des fibres kératiniques humaines, sèches ou humides, à décolorer, la composition de décoloration prête à l'emploi selon l'invention ; à la laisser agir pendant un temps de pause suffisant pour obtenir la décoloration recherchée ; à éliminer la composition par un rinçage à l'eau, suivi d'un lavage avec un shampooing, puis éventuellement d'un séchage.

Habituellement, le temps de pause varie entre 1 et 60 minutes, plus préférentiellement entre 10 et 50 minutes.

L'invention a enfin pour objet un dispositif à plusieurs compartiments, ou "kit", pour la mise en oeuvre du procédé de décoloration des fibres kératiniques humaines.
Ce dispositif comporte au moins deux compartiments dont l'un contient une composition de décoloration anhydre sous forme de pâte comprenant au moins un sel peroxygéné, au moins un agent alcalin et de 15 à 35 % en poids d'au moins un liquide inerte organique ; et l'autre contient une émulsion huile dans eau de peroxyde d'hydrogène, comprenant au moins un tensioactif non ionique et/ou anionique et au moins un copolymère comprenant au moins un motif provenant d'un acide (méth)acrylique salifié ou non et au moins un motif provenant d'un ester d'acide (méth)acrylique avec un alcool gras en C₈-C₃₀, saturé ou insaturé, linéaire ou ramifié, éthoxylé.

Des exemples non limitatifs de la présente invention vont maintenant être présentés.

### Exemples

### Composition de décoloration A sous forme de pâte anhydre

On prépare la composition A suivante :

| | Quantités (g%) |
|---|---|
| Persulfate de Potassium | 41,3 |
| Disilicate de Sodium | 18 |
| Chlorure d'ammonium | 2,2 |
| Sulfate d'ammonium | 2 |
| EDTA | 0,2 |
| Copolymère hexaméthyl di-isocyanate/polyéthylèneglycol à terminaison α et ω stéaryl polyoxyéthylène (SER-AD FX 1100 - Servo Delden) | 0,5 |
| Carboxyméthyl amidon de pomme de terre / sel de sodium faiblement réticulé (PRIMOJEL de AVEBE) | 1 |
| Gomme de Guar | 0,5 |
| Gomme xanthane | 2 |
| Oxyde de titane | 1 |
| Cétostéaryl sulfate de sodium | 2 |
| Lauryl sulfate de sodium | 2 |
| Stéarate de magnésium | 2 |
| Ultramarine | 0,5 |
| Myristate d'isopropyle (Cognis) | 24,2 |
| Silice pyrogénée à caractère hydrophile, (Aerosil 300 - Degussa Hüls) | 0,6 |

### Compositions oxydantes (Emulsions H/E)

On prépare les émulsions suivantes :

| | B | C | D | E |
|---|---|---|---|---|
| | (g%) | (g%) | (g%) | (g%) |
| Alcool cétylique | 8 | 8 | 10 | 8 |
| Alcool cétylique (20 OE) | 2 | 2 | 2 | 4 |
| Glycérine | 0,5 | 0,5 | 0,5 | 0,5 |
| Copolymère acrylate / beheneth-25 métacrylate (Aculyn 28 -ISP/Rohm & Haas) | 2 | / | / | / |
| Tétrasodium pyrophosphate | 0,04 | 0,04 | 0,04 | 0,04 |
| Etidronate de sodium | 0,2 | 0,2 | 0,2 | 0,2 |
| Eau oxygénée à 50 % | 18 | 18 | 18 | 18 |
| Acide phosphorique à 85 % | Qs pH 3 | Qs pH 3 | Qs pH 3 | Qs pH 3 |
| Eau | Qsp 100g | Qsp 100g | Qsp 100g | Qsp 100g |

### Compositions aqueuses de décoloration prêtes à l'emploi

- COMP A/B : 20g de composition de décoloration A + 30g de composition oxydante B
- COMP A/C : 20g de composition de décoloration A + 30g de composition oxydante C
- COMP A/D : 20g de composition de décoloration A + 30g de composition oxydante D
- COMP A/E : 20g de composition de décoloration A + 30g de composition oxydante E

Les compositions sont obtenues par mélange.

### Evaluation de la rapidité de mélange des compositions aqueuses de décoloration prêtes à l'emploi :

- Evaluation du temps de mélange en secondes (cf. via un chronomètre)
- Déclenchement du chronomètre au premier coup de spatule
- Arrêt du chronomètre lorsque les mélanges sont lisses et homogènes
- Evaluation par un panel de 5 personnes

| | COMP A/B | COMP A/C | COMP A/D | COMP A/E |
|---|---|---|---|---|
| Evaluation du temps de mélange / Moyenne (seconde) | 65 | 94 | 84 | 91 |
| Ecart-type (secondes) | 6 | 6 | 6 | 5 |

Le mélange des compositions A et B (COMP A/B) selon l'invention est significativement plus rapide à réaliser.

De plus, la composition de décoloration prête à l'emploi COMP A/B s'applique facilement et rapidement. Elle présente une très bonne adhérence. Elle ne coule pas hors des zones de chevelure que l'on souhaite décolorer. Elle permet enfin une décoloration puissante et homogène, tout en offrant de très bonnes propriétés cosmétiques.

## Revendications

1. Composition de décoloration de fibres kératiniques humaines, et en particulier des cheveux, prête à l'emploi, susceptible d'être obtenue en mélangeant avant emploi :
i) une composition de décoloration anhydre sous forme de pâte comprenant au moins un sel peroxygéné, au moins un agent alcalin et de 15 à 35 % en poids d'au moins un liquide inerte organique, avec,
ii) une émulsion huile dans eau de peroxyde d'hydrogène, comprenant au moins un tensioactif non ionique et/ou anionique et au moins un copolymère comprenant au moins un motif provenant d'un acide (méth)acrylique salifié ou non et au moins un motif provenant d'un ester d'acide (méth)acrylique avec un alcool gras en C₈-C₃₀, saturé ou insaturé, linéaire ou ramifié, éthoxylé.

2. Composition selon la revendication précédente, **caractérisée en ce que** le liquide inerte organique comprend au moins un composé choisi parmi les polydécènes, les mono- et poly- esters d'acides carboxyliques, les mono- ou poly- esters de sucres d'acides en C₈-C₃₀, les éthers cycliques, les esters cycliques, les huiles silicones, les huiles minérales, les huiles végétales, ou leurs mélanges.

3. Composition selon l'une quelconque des revendications précédentes, **caractérisée en que** le liquide inerte est choisi parmi les esters d'acides en C₈-C₃₀ et d'un monoalcool saturé, linéaire ou ramifié, en C₃-C₆.

4. Composition selon l'une quelconque des revendications précédentes, **caractérisée en que** le sel peroxygéné est choisi parmi les persulfates, perborates, percarbonates et peroxydes de métaux alcalins et alcalino-terreux, ou leurs mélanges.

5. Composition selon la revendication précédente, **caractérisée en que** le sel peroxygéné est choisi parmi les persulfates de sodium et de potassium, seuls ou en mélange.

6. Composition selon l'une quelconque des revendications précédentes, **caractérisée en ce que** la teneur en sel peroxygéné dans la composition de décoloration anhydre est comprise entre 10 et 70 % en poids, de préférence entre 20 et 60 % en poids.

7. Composition selon l'une quelconque des revendications précédentes, **caractérisée en ce que** la teneur en sel peroxygéné est comprise entre 5 et 35 % en poids de la composition prête à l'emploi, de préférence entre 10 et 30 % en poids de la composition prête à l'emploi.

8. Composition selon l'une quelconque des revendications précédentes, **caractérisée en ce que** l'agent alcalin est chois parmi l'urée ; les sels d'ammonium ; les silicates, les phosphates, les carbonates de métaux alcalins ou alcalino-terreux, seuls ou en mélange.

9. Composition selon l'une quelconque des revendications précédentes, **caractérisée en ce que** la teneur en agent alcalin dans la composition de décoloration anhydre est comprise entre 0,01 et 40 % en poids, de préférence entre 0,1 et 30 % en poids.

10. Composition selon l'une quelconque des revendications précédentes, **caractérisée en ce que** la teneur en agent alcalin est comprise entre 0,005 et 20 % en poids de la composition prête à l'emploi, de préférence entre 0,05 et 15 % en poids de la composition prête à l'emploi.

11. Composition selon l'une quelconque des revendications précédentes, **caractérisée en ce que** la composition de décoloration anhydre comprend au moins un agent épaississant hydrosoluble dépourvu de chaîne hydrophobe.

12. Composition selon la revendication 11, **caractérisée en ce que** la teneur en agent épaississant dans la composition de décoloration anhydre représente 0,01 à 30 % en poids de la composition prête à l'emploi.

13. Composition selon l'une quelconque des revendications précédentes, **caractérisée en ce que** la composition de décoloration anhydre comprend au moins un tensioactif non ionique, anionique, amphotère, zwitterionique ou cationique, ou leurs mélanges.

14. Composition selon l'une quelconque des revendications précédentes, **caractérisée en ce que** la composition de décoloration anhydre comprend au moins un polymère amphiphile comportant au moins une chaîne hydrophobe, de préférence différent de celui ou ceux présents dans l'émulsion huile dans eau.

15. Composition selon la revendication 14, **caractérisée en ce que** la teneur en polymère amphiphile comportant au moins une chaîne hydrophobe représente 0,01 à 30 % en poids de la composition prête à l'emploi.

16. Composition selon l'une quelconque des revendications précédentes, **caractérisée en ce que** la composition de décoloration anhydre comprend moins de 1 % en poids d'eau, et de préférence moins de 0,5 % en poids d'eau.

17. Composition selon l'une quelconque des revendications précédentes, **caractérisée en ce que** le copolymère présent dans l'émulsion huile dans eau comprend au moins un motif provenant d'un ester d'acide (méth)acrylique et d'un alcool saturé en C₁-C₆, de préférence en C₁-C₂ éthoxylé.

18. Composition selon la revendication précédente, **caractérisée en ce que** ledit copolymère comprend au moins un motif dérivé d'acide (méth)acrylique, au moins un motif dérivé d'acrylate d'éthyle et au moins un motif dérivé de (méth)acrylate de béhényle éthoxylé, de préférence comprenant 25 moles d'oxyde d'éthylène.

19. Composition selon l'une quelconque des revendications précédentes, **caractérisée en ce que** la teneur en copolymère représente 0,005 à 15 % en poids de la composition prête à l'emploi, plus particulièrement de 0,05 à 7,5 % en poids de la composition prête à l'emploi, de préférence de 0,1 à 5 % en poids de la composition prête à l'emploi.

20. Composition selon l'une quelconque des revendications précédentes, **caractérisée en ce que** l'émulsion huile dans eau comprend au moins un tensioactif non ionique polyalcoxylé ou polyglycérolé, de préférence polyéthoxylé.

21. Composition selon l'une quelconque des revendications 13 ou 20, **caractérisée en ce que** la teneur totale en tensioactif représente 0,05 et 30 % en poids de la composition prête à l'emploi, de préférence entre 0,1 et 20 % en poids de la composition prête à l'emploi.

22. Composition selon l'une quelconque des revendications précédentes, **caractérisée en ce que** la phase huile de l'émulsion huile dans eau comprend au moins un alcool gras.

23. Composition selon l'une quelconque des revendications précédentes, **caractérisée en ce que** la teneur en peroxyde d'hydrogène dans la composition prête à l'emploi représente de 1 à 12 % en titre en peroxyde d'hydrogène, plus particulièrement de 2 à 9% en titre en peroxyde d'hydrogène, de préférence de 2 à 6 % en titre en peroxyde d'hydrogène.

24. Composition selon l'une quelconque des revendications précédentes, **caractérisée en ce que** le pH de l'émulsion huile dans eau est compris entre 1 et 6, de préférence entre 1 et 4.

25. Composition selon l'une quelconque des revendications précédentes, **caractérisée en ce que** le pH de la composition prête à l'emploi est compris entre 4 et 12, plus particulièrement entre 7 et 11,5, de préférence entre 8 et 11.

26. Procédé de préparation de la composition de décoloration de fibres kératiniques humaines, et en particulier des cheveux, prête à l'emploi, selon l'une quelconque des revendications 1 à 25, **caractérisé en ce que** l'on mélange avant emploi :
i) une composition de décoloration anhydre sous forme de pâte comprenant au moins un sel peroxygéné, au moins un agent alcalin et de 15 à 35 % en poids d'au moins un liquide inerte organique, avec,
ii) une émulsion huile dans eau de peroxyde d'hydrogène, comprenant au moins un tensioactif non ionique et/ou anionique et au moins un copolymère comprenant au moins un motif provenant d'un acide (méth)acrylique salifié ou non et au moins un motif provenant d'un ester d'acide (méth)acrylique avec un alcool gras en C₈-C₃₀, saturé ou insaturé, linéaire ou ramifié, éthoxylé.

27. Procédé de décoloration de fibres kératiniques humaines, en particulier des cheveux, consistant à appliquer la composition de décoloration prête à l'emploi obtenue selon l'une quelconque des revendications 1 à 25, sur la zone des fibres kératiniques humaines, sèches ou humides, à décolorer ; à la laisser agir pendant un temps de pause suffisant pour obtenir la décoloration recherchée ; à éliminer la composition par un rinçage à l'eau, suivi d'un lavage avec un shampooing, puis éventuellement d'un séchage.

28. Procédé de décoloration selon la revendication précédente, **caractérisé en ce que** le temps de pause varie entre 1 et 60 minutes, plus préférentiellement entre 10 et 50 minutes.

29. Dispositif à plusieurs compartiments, ou "kit", pour la mise en oeuvre du procédé selon l'une des revendications 27 ou 28, **caractérisé par le fait qu'**il comporte au moins deux compartiments dont l'un contient une composition de décoloration anhydre sous forme de pâte comprenant au moins un sel peroxygéné, au moins un agent alcalin et de 15 à 35 % en poids d'au moins un liquide inerte organique ; et l'autre contient une émulsion huile dans eau de peroxyde d'hydrogène, comprenant au moins un tensioactif non ionique et/ou anionique et au moins un copolymère comprenant au moins un motif provenant d'un acide (méth)acrylique salifié ou non et au moins un motif provenant d'un ester d'acide (méth)acrylique avec un alcool gras en C₈-C₃₀, saturé ou non, linéaire ou ramifié, éthoxylé.

## Claims

1. Ready-to-use composition for bleaching human keratin fibres, and in particular the hair, which can be obtained by mixing before use:
i) an anhydrous bleaching composition in paste form comprising at least one peroxygenated salt, at least one alkaline agent and from 15% to 35% by weight of at least one inert organic liquid, with
ii) a hydrogen peroxide oil-in-water emulsion comprising at least one nonionic and/or anionic surfactant and at least one copolymer comprising at least one unit derived from a salified or non-salified (meth)acrylic acid and at least one unit derived from an ester of (meth)acrylic acid with a saturated or unsaturated, linear or branched, ethoxylated C₈-C₃₀ fatty alcohol.

2. Composition according to the preceding claim, **characterized in that** the inert organic liquid comprises at least one compound chosen from polydecenes, carboxylic acid monoesters and polyesters, sugar monoesters or polyesters of C₈-C₃₀ acids, cyclic ethers, cyclic esters, silicone oils, mineral oils and plant oils, or mixtures thereof.

3. Composition according to either of the preceding claims, **characterized in that** the inert liquid is chosen from C₈-C₃₀ acid esters of a saturated, linear or branched C₃-C₆ monoalcohol.

4. Composition according to any one of the preceding claims, **characterized in that** the peroxygenated salt is chosen from alkali metal and alkaline-earth metal persulphates, perborates, percarbonates and peroxides, or mixtures thereof.

5. Composition according to the preceding claim, **characterized in that** the peroxygenated salt is chosen from sodium persulphate and potassium persulphate, alone or as a mixture.

6. Composition according to any one of the preceding claims, **characterized in that** the content of peroxygenated salt in the anhydrous bleaching composition is between 10% and 70% by weight and preferably between 20% and 60% by weight.

7. Composition according to any one of the preceding claims, **characterized in that** the content of peroxygenated salt is between 5% and 35% by weight of the ready-to-use composition and preferably between 10% and 30% by weight of the ready-to-use composition.

8. Composition according to any one of the preceding claims, **characterized in that** the alkaline agent is chosen from urea; ammonium salts; alkali metal or alkaline-earth metal silicates, phosphates and carbonates, alone or as a mixture.

9. Composition according to any one of the preceding claims, **characterized in that** the content of alkaline agent in the anhydrous bleaching composition is between 0.01% and 40% by weight and preferably between 0.1% and 30% by weight.

10. Composition according to any one of the preceding claims, **characterized in that** the content of alkaline agent is between 0.005% and 20% by weight of the ready-to-use composition and preferably between 0.05% and 15% by weight of the ready-to-use composition.

11. Composition according to any one of the preceding claims, **characterized in that** the anhydrous bleaching composition comprises at least one water-soluble thickener not containing a hydrophobic chain.

12. Composition according to Claim 11, **characterized in that** the content of thickener in the anhydrous bleaching composition represents 0.01% to 30% by weight of the ready-to-use composition.

13. Composition according to any one of the preceding claims, **characterized in that** the anhydrous bleaching composition comprises at least one nonionic, anionic, amphoteric, zwitterionic or cationic surfactant, or mixtures thereof.

14. Composition according to any one of the preceding claims, **characterized in that** the anhydrous bleaching composition comprises at least one amphiphilic polymer comprising at least one hydrophobic chain, which is preferably different from the polymer(s) present in the oil-in-water emulsion.

15. Composition according to Claim 14, **characterized in that** the content of amphiphilic polymer comprising at least one hydrophobic chain represents 0.01% to 30% by weight of the ready-to-use composition.

16. Composition according to any one of'the preceding claims, **characterized in that** the anhydrous bleaching composition comprises less than 1% by weight of water and preferably less than 0.5% by weight of water.

17. Composition according to any one of the preceding claims, **characterized in that** the copolymer present in the oil-in-water emulsion comprises at least one unit derived from an ester of (meth)acrylic acid and of a saturated C₁-C₆, and preferably C₁-C₂, ethoxylated alcohol.

18. Composition according to the preceding claim, **characterized in that** the said copolymer comprises at least one unit derived from (meth)acrylic acid, at least one unit derived from ethyl acrylate and at least one unit derived from ethoxylated behenyl (meth)acrylate, preferably comprising 25 mol of ethylene oxide.

19. Composition according to any one of the preceding claims, **characterized in that** the copolymer content represents 0.005% to 15% by weight of the ready-to-use composition, more particularly from 0.05% to 7.5% by weight of the ready-to-use composition and preferably from 0.1% to 5% by weight of the ready-to-use composition.

20. Composition according to any one of the preceding claims, **characterized in that** the oil-in-water emulsion comprises at least one polyalkoxylated or polyglycerolated, preferably polyethoxylated, nonionic surfactant.

21. Composition according to either of Claims 13 and 20, **characterized in that** the total content of surfactant represents between 0.05% and 30% by weight of the ready-to-use composition and preferably between 0.1% and 20% by weight of the ready-to-use composition.

22. Composition according to any one of the preceding claims, **characterized in that** the oil phase of the oil-in-water emulsion comprises at least one fatty alcohol.

23. Composition according to any one of the preceding claims, **characterized in that** the content of hydrogen peroxide in the ready-to-use composition represents from 1% to 12% as hydrogen peroxide titre, more particularly from 2% to 9% as hydrogen peroxide titre and preferably from 2% to 6% as hydrogen peroxide titre.

24. Composition according to any one of the preceding claims, **characterized in that** the pH of the oil-in-water emulsion is between 1 and 6 and preferably between 1 and 4.

25. Composition according to any one of the preceding claims, **characterized in that** the pH of the ready-to-use composition is between 4 and 12, more particularly between 7 and 11.5 and preferably between 8 and 11.

26. Process for preparing the ready-to-use composition for bleaching human keratin fibres, and in particular the hair, according to any one of Claims 1 to 25, **characterized in that,** before use:
i) an anhydrous bleaching composition in paste form comprising at least one peroxygenated salt, at least one alkaline agent and from 15% to 35% by weight of at least one inert organic liquid, is mixed with
ii) a hydrogen peroxide oil-in-water emulsion comprising at least one nonionic and/or anionic surfactant and at least one copolymer comprising at least one unit derived from a salified or non-salified (meth)acrylic acid and at least one unit derived from an ester of (meth)acrylic acid with a saturated or unsaturated, linear or branched, ethoxylated C₈-C₃₀ fatty alcohol.

27. Process for bleaching human keratin fibres, in particular the hair, which consists in applying the ready-to-use bleaching composition obtained according to any one of Claims 1 to 25 to the area of wet or dry human keratin fibres to be bleached; leaving the composition to act for a leave-in time that is sufficient to obtain the desired bleaching result; removing the composition by rinsing with water, followed by washing with shampoo and then optionally drying.

28. Bleaching process according to the preceding claim, **characterized in that** the leave-in time ranges between 1 and 60 minutes and more preferably between 10 and 50 minutes.

29. Multi-compartment device or "kit" for implementing the process according to either of Claims 27 and 28, **characterized in that** it comprises at least two compartments, one of which contains an anhydrous bleaching composition in paste form comprising at least one peroxygenated salt, at least one alkaline agent and from 15% to 35% by weight of at least one inert organic liquid; and the other contains a hydrogen peroxide oil-in-water emulsion comprising at least one nonionic and/or anionic surfactant and at least one copolymer comprising at least one unit derived from a salified or non-salified (meth)acrylic acid and at least one unit derived from an ester of (meth)acrylic acid with a saturated or unsaturated, linear or branched, ethoxylated C₈-C₃₀ fatty alcohol.

## Patentansprüche

1. Gebrauchsfertige Zusammensetzung zum Entfärben von menschlichen Keratinfasern und insbesondere Haaren, die erhältlich ist, indem vor der Anwendung vermischt werden:
(i) eine wasserfreie Zusammensetzung zum Entfärben in der Form einer Paste, die mindestens ein Peroxysalz, mindestens eine Base und 15 bis 35 Gew.-% mindestens einer inerten organischen Flüssigkeit enthält, mit
(ii) einer Öl-in-Wasser-Emulsion mit Wasserstoffperoxid, die mindestens einen nichtionischen und/oder anionischen grenzflächenaktiven Stoff und mindestens ein Copolymer enthält, das mindestens eine Einheit, die von (Meth)acrylsäure, die gegebenenfalls als Salz vorliegt, abgeleitet ist, und mindestens eine Einheit umfasst, die von einem Ester der (Meth)acrylsäure und eines gesättigten oder ungesättigten, linearen oder verzweigten, ethoxylierten C₈₋₃₀-Fettalkohols stammt.

2. Zusammensetzung nach dem vorhergehenden Anspruch, **dadurch gekennzeichnet, dass** die inerte organische Flüssigkeit mindestens eine Verbindung umfasst, die unter den Polydecenen, Mono- und Polyestern von Carbonsäuren, Mono- oder Polyestern von Zuckern und C₈₋₃₀-Fettsäuren, cyclischen Ethern, cyclischen Estern, Siliconölen, Mineralölen, pflanzlichen Ölen oder deren Gemischen ausgewählt ist.

3. Zusammensetzung nach einem der vorhergehenden Ansprüche, **dadurch gekennzeichnet, dass** die inerte Flüssigkeit unter den Estern von C₈₋₃₀-Säuren und eines gesättigten, linearen oder verzweigten C₃₋₆-Monoalkohols ausgewählt ist.

4. Zusammensetzung nach einem der vorhergehenden Ansprüche, **dadurch gekennzeichnet, dass** das Peroxysalz unter den Persulfaten, Perboraten, Percarbonaten und Peroxiden von Alkalimetallen und Erdalkalimetallen oder deren Gemischen ausgewählt ist.

5. Zusammensetzung nach dem vorhergehenden Anspruch, **dadurch gekennzeichnet, dass** das Peroxysalz einzeln oder in Form eines Gemisches unter Natriumpersulfat und Kaliumpersulfat ausgewählt ist.

6. Zusammensetzung nach einem der vorhergehenden Ansprüche, **dadurch gekennzeichnet, dass** der Mengenanteil des Peroxysalzes in der wasserfreien Zusammensetzung zum Entfärben im Bereich von 10 bis 70 Gew.-% und vorzugsweise 20 bis 60 Gew.-% liegt.

7. Zusammensetzung nach einem der vorhergehenden Ansprüche, **dadurch gekennzeichnet, dass** der Mengenanteil des Peroxysalzes im Bereich von 5 bis 35 Gew.-% der gebrauchsfertigen Zusammensetzung und vorzugsweise im Bereich von 10 bis 30 Gew.-% der gebrauchsfertigen Zusammensetzung liegt.

8. Zusammensetzung nach einem der vorhergehenden Ansprüche, **dadurch gekennzeichnet, dass** die Base einzeln oder in Form eines Gemisches unter Harnstoff; Ammoniumsalzen; Silicaten, Phosphaten oder Carbonaten von Alkali- oder Erdalkalimetallen ausgewählt ist.

9. Zusammensetzung nach einem der vorhergehenden Ansprüche, **dadurch gekennzeichnet, dass** der Mengenanteil der Base in der wasserfreien Zusammensetzung zum Entfärben im Bereich von 0,01 bis 40 Gew.-% und vorzugsweise 0,1 bis 30 Gew.-% liegt.

10. Zusammensetzung nach einem der vorhergehenden Ansprüche, **dadurch gekennzeichnet, dass** der Mengenanteil der Base im Bereich von 0,005 bis 20 Gew.-% der gebrauchsfertigen Zusammensetzung und vorzugsweise im Bereich von 0,05 bis 15 Gew.-% der gebrauchsfertigen Zusammensetzung liegt.

11. Zusammensetzung nach einem der vorhergehenden Ansprüche, **dadurch gekennzeichnet, dass** die wasserfreie Zusammensetzung zum Entfärben mindestens ein wasserlösliches Verdickungsmittel ohne hydrophobe Kette enthält.

12. Zusammensetzung nach Anspruch 11, **dadurch gekennzeichnet, dass** der Mengenanteil des Verdickungsmittels in der wasserfreien Zusammensetzung zum Entfärben 0,01 bis 30 Gew.-% der gebrauchsfertigen Zusammensetzung beträgt.

13. Zusammensetzung nach einem der vorhergehenden Ansprüche, **dadurch gekennzeichnet, dass** die wasserfreie Zusammensetzung zum Entfärben mindestens einen nichtionischen, anionischen, amphoteren, zwitterionischen oder kationischen grenzflächenaktiven Stoff oder deren Gemische enthält.

14. Zusammensetzung nach einem der vorhergehenden Ansprüche, **dadurch gekennzeichnet, dass** die wasserfreie Zusammensetzung zum Entfärben mindestens ein amphiphiles Polymer enthält, das mindestens eine hydrophobe Kette aufweist und vorzugsweise von dem oder den in der Öl-in-Wasser-Emulsion enthaltenen Polymer(en) verschieden ist.

15. Zusammensetzung nach Anspruch 14, **dadurch gekennzeichnet, dass** der Mengenanteil des amphiphilen Polymers, das mindestens eine hydrophobe Kette enthält, 0,01 bis 30 Gew.-% der gebrauchsfertigen Zusammensetzung ausmacht.

16. Zusammensetzung nach einem der vorhergehenden Ansprüche, **dadurch gekennzeichnet, dass** die wasserfreie Zusammensetzung zum Entfärben weniger als 1 Gew.-% Wasser und vorzugsweise weniger als 0,5 Gew.-% Wasser enthält.

17. Zusammensetzung nach einem der vorhergehenden Ansprüche, **dadurch gekennzeichnet, dass** das in der Öl-in-Wasser-Emulsion enthaltene Copolymer mindestens eine Einheit umfasst, die von einem Ester der (Meth)acrylsäure und eines gesättigten C₁₋₆-Alkohols und vorzugsweise eines ethoxylierten C₁₋₂-Alkohols abgeleitet ist

18. Zusammensetzung nach dem vorhergehenden Anspruch, **dadurch gekennzeichnet, dass** das Copolymer mindestens eine Einheit, die von (Meth)acrylsäure abgeleitet ist, mindestens eine Einheit, die von Ethylacrylat abgeleitet ist, und mindestens eine Einheit umfasst, die von Behenyl(meth)acrylat abgeleitet ist und ethoxyliert ist und vorzugsweise 25 mol Ethylenoxid umfasst.

19. Zusammensetzung nach einem der vorhergehenden Ansprüche, **dadurch gekennzeichnet, dass** der Mengenanteil des Copolymers 0,005 bis 15 Gew.-% der gebrauchsfertigen Zusammensetzung, insbesondere 0,05 bis 7,5 Gew.-% der gebrauchsfertigen Zusammensetzung und vorzugsweise 0,1 bis 5 Gew.-% der gebrauchsfertigen Zusammensetzung ausmacht.

20. Zusammensetzung nach einem der vorhergehenden Ansprüche, **dadurch gekennzeichnet, dass** die Öl-in-Wasser-Emulsion mindestens einen polyalkoxylierten oder mehrfach mit Glycerin veretherten und vorzugsweise polyethoxylierten nichtionischen grenzflächenaktiven Stoff enthält.

21. Zusammensetzung nach einem der Ansprüche 13 oder 20, **dadurch gekennzeichnet, das** die Gesamtmenge des grenzflächenaktiven Stoffes 0,05 bis 30 Gew.-% der gebrauchsfertigen Zusammensetzung und vorzugsweise 0,1 bis 20 Gew.-% der gebrauchsfertigen Zusammensetzung ausmacht.

22. Zusammensetzung nach einem der vorhergehenden Ansprüche, **dadurch gekennzeichnet, dass** die Ölphase der Öl-in-Wasser-Emulsion mindestens einen Fettalkohol enthält.

23. Zusammensetzung nach einem der vorhergehenden Ansprüche, **dadurch gekennzeichnet, dass** der als Wasserstoffperoxid-Titer ausgedrückte prozentuale Anteil des Wasserstoffperoxids in der gebrauchsfertigen Zusammensetzung 1 bis 12 %, insbesondere 2 bis 9 % und vorzugsweise 2 bis 6 % beträgt.

24. Zusammensetzung nach einem der vorhergehenden Ansprüche, **dadurch gekennzeichnet, dass** der pH-Wert der Öl-in-Wasser-Emulsion im Bereich von 1 bis 6 und vorzugsweise im Bereich von 1 bis 4 liegt.

25. Zusammensetzung nach einem der vorhergehenden Ansprüche, **dadurch gekennzeichnet, dass** der pH-Wert der gebrauchsfertigen Zusammensetzung im Bereich von 4 bis 12, insbesondere 7 bis 11,5 und vorzugsweise 8 bis 11 liegt.

26. Verfahren zur Herstellung einer gebrauchsfertigen Zusammensetzung zum Entfärben von menschlichen Keratinfasern und insbesondere Haaren nach einem der Ansprüche 1 bis 25, **dadurch gekennzeichnet, dass** vor der Anwendung vermischt werden:
(i) eine wasserfreie Zusammensetzung zum Entfärben in der Form einer Paste, die mindestens ein Peroxysalz, mindestens eine Base und 15 bis 35 Gew.-% mindestens einer inerten organischen Flüssigkeit enthält, mit
(ii) einer Öl-in-Wasser-Emulsion mit Wasserstoffperoxid, die mindestens einen nichtionischen und/oder anionischen grenzflächenaktiven Stoff und mindestens ein Copolymer enthält, das mindestens eine Einheit, die von (Meth)acrylsäure, die gegebenenfalls als Salz vorliegt, abgeleitet ist, und mindestens eine Einheit umfasst, die von einem Ester der (Meth)acrylsäure und eines gesättigten oder ungesättigten, linearen oder verzweigten, ethoxylierten C₈₋₃₀-Alkohols stammt.

27. Verfahren zum Entfärben vom menschlichen Keratinfasern und insbesondere Haaren, das darin besteht, die gebrauchsfertige Zusammensetzung zum Entfärben nach einem der Ansprüche 1 bis 25 auf den Bereich der feuchten oder trockenen menschlichen Keratinfasern aufzutragen, der entfärbt werden soll; während einer Einwirkzeit, die ausreichend ist, um die gewünschte Entfärbung zu erhalten, einwirken zu lassen; die Zusammensetzung durch Spülen mit Wasser und anschließende Waschen mit einem Haarwaschmittel zu entfernen und dann gegebenenfalls zu trocknen.

28. Verfahren zum Entfärben nach dem vorhergehenden Anspruch, **dadurch gekennzeichnet, dass** die Einwirkzeit im Bereich von 1 bis 60 min und vorzugsweise 10 bis 50 min liegt.

29. Vorrichtung mit mehreren Abteilungen oder "Kit" für die Durchführung des Verfahrens nach einem der Ansprüche 27 oder 28, **dadurch gekennzeichnet, dass** sie mindestens zwei Abteilungen aufweist, wobei eine Abteilung eine wasserfreie Zusammensetzung zum Entfärben in der Form einer Paste enthält, die mindestens ein Peroxysalz, mindestens eine Base und 15 bis 35 Gew.-% mindestens einer inerten organischen Flüssigkeit enthält, und die andere Abteilung eine Öl-in-Wasser-Emulsion mit Wasserstoffperoxid enthält, die mindestens einen nichtionischen und/oder anionischen grenzflächenaktiven Stoff und mindestens ein Copolymer enthält, das mindestens eine Einheit, die von (Meth)acrylsäure, die gegebenenfalls als Salz vorliegt, abgeleitet ist, und mindestens eine Einheit umfasst, die von einem Ester der (Meth)acrylsäure und eines gesättigten oder ungesättigten, linearen oder verzweigten, ethoxylierten C₈₋₃₀-Alkohols stammt.
